# FASCICULE DE BREVET EUROPEEN

(11) **EP 1 032 353 B1**
(45) Date de publication et mention de la délivrance du brevet: **05.09.2001**
(21) Numéro de dépôt: 98954562.9
(22) Date de dépôt: 12.11.1998
(51) Int. Cl.: A61K 7/00, A61K 7/02, A61K 7/021

(54) **COMPOSITION COSMETIQUE COMPRENANT UN COMPOSE DE TYPE INDIGOIDE**
KOSMETISCHE ZUSAMMENSETZUNG ENTHALTEND EINE INDIGOTYP-VERBINDUNG
COSMETIC COMPOSITION COMPRISING A COMPOUND SUCH AS INDIGOID

(30) Priorité: 21.11.1997 FR 9714656
(43) Date de publication de la demande: 06.09.2000
(73) Titulaire: L'OREAL, 75008 Paris (FR)
(72) Inventeur: PHILIPPE, Michel, F-91320 Wissous (FR); HENRION, Jean-Christophe, F-93500 Pantin (FR); GUILLIER-BERTEUIL, Nathalie, F-91800 Brunoy (FR)
(74) Mandataire: Dodin, Catherine
(86) Numéro de dépôt international: FR9802411
(87) Numéro de publication internationale: WO9926586

(56) Documents cités:
- DE-A- 4 427 888
- STN, Serveur de Bases de Données, XP002077029 & GOELTNER ET AL.: "2,2'-Binaphtylidene-1,1'-diones: color and structure" LIEBIGS ANN. CHEM., vol. 10, 1991, pages 1085-1089, DE cité dans la demande
- STN, Serveur de Bases de Données, XP002077030

## Description

La présente invention a trait à l'utilisation en cosmétique de composés de type indigoïdes, notamment pour apporter de la couleur à des compositions cosmétiques.

Les compositions cosmétiques, et notamment les compositions de maquillage telles que les poudres libres ou compactes, les fonds de teint, les fards à joues ou à paupières, les rouges à lèvres ou les vernis à ongles, sont constituées d'un véhicule approprié et de différents agents colorants destinés à conférer une certaine couleur auxdites compositions avant et/ou après leur application sur la peau, les muqueuses et/ou les phanères telles que les ongles, les cils ou les cheveux.
Pour créer des couleurs, on utilise aujourd'hui une gamme d'agents colorants assez limitée, parmi lesquels on peut citer des composés généralement insolubles dans les milieux aqueux et organiques tels que des laques organiques. des pigments minéraux ou des pigments nacrés.
Les pigments et laques utilisés dans le domaine du maquillage sont d'origine et de nature chimique très diverses. Leurs propriétés physico-chimiques, notamment granulométrie, surface spécifique, densité, etc., sont donc très différentes. Ces différences se traduisent par des variations de comportement : leur facilité de mise en oeuvre, de dispersion dans le milieu; leur stabilité à la lumière, à la température; leurs propriétés mécaniques.
Ainsi, les pigments minéraux, en particulier les oxydes minéraux tels que les oxydes de fer, sont très stables à la lumière et au pH, mais donnent des couleurs plutôt ternes, fades et pâles. Il est donc nécessaire d'en introduire une grande quantité dans les formulations cosmétiques pour obtenir un trait suffisamment saturé. Ce fort pourcentage de particules minérales peut néanmoins affecter la brillance de la composition.
Pour obtenir des effets colorés, on peut encore employer des pigments nacrés de couleurs variées, mais jamais très intenses, qui permettent d'obtenir des effets irisés mais le plus souvent assez faibles.
Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments minéraux usuels pour apporter un reflet temporaire aux cheveux, mais les nuances obtenues par cette coloration restent assez ternes, trop uniformes et peu ludiques.
Dans le domaine du maquillage, seules les laques organiques permettaient jusqu'à présent d'obtenir des couleurs vives et intenses. Cependant, la plupart des laques organiques présentent une très mauvaise tenue à la lumière, qui se traduit par une atténuation très nette de leur couleur dans le temps. Elles peuvent également être instables à la température et/ou au pH. De plus, certaines laques génèrent un dégorgement trop important. c'est-à-dire qu'elles présentent l'inconvénient de tacher le support sur lequel elles sont appliquées. Ainsi, ceci peut avoir pour conséquence de tâcher les lentilles oculaires dans le cas des eye-liners ou des mascaras, ou de laisser une coloration sur la peau ou les ongles après démaquillage dans le cas des rouges à lèvres ou des vernis à ongles. Enfin, l'instabilité des laques est encore aggravée lorsqu'elles sont associées à des pigments photoréactifs comme le dioxyde de titane. Or ces pigments sont très largement utilisés dans le maquillage, notamment pour la protection contre le rayonnement UV. Par conséquent, l'utilisation des laques organiques en cosmétique est assez limitée, ce qui a pour conséquence une limitation des teintes réalisables.

Ainsi il subsiste le besoin de disposer d'agents colorants susceptibles d'être utilisés en cosmétique et permettant d'obtenir une coloration adéquate des compositions et du film de maquillage obtenu, lesdits agents colorants ne devant pas dégorger sur le support sur lequel lesdites compositions sont déposées.

Après de nombreuses recherches, la demanderesse a mis en évidence que l'utilisation d'une famille bien précise de composés organiques permettait d'obtenir un tel résultat.

Ainsi l'invention a pour objet une composition cosmétique comprenant, dans un milieu cosmétiquement acceptable, au moins un composé de formule (I): 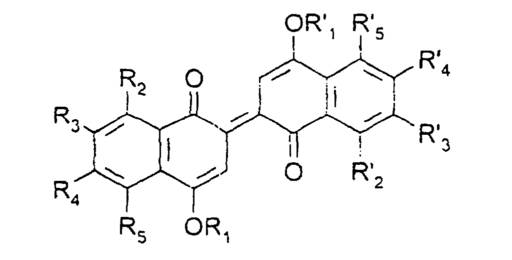 dans laquelle
* R1 et R'1 sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes;
* R2, R'2, R3, R'3, R4, R'4, R5, R'5 sont. indépendamment les uns des autres, choisis parmi un atome d'hydrogène, un radical halogéné, un radical hydroxyle, un radical alkyle, alkyloxy, acyle ou acyloxy, linéaire ou ramifié, saturé ou insaturé, ayant 1 à 6 atomes de carbone.

L'invention a également pour objet l'utilisation d'au moins un composé de formule (I) pour enrober au moins partiellement des particules substrats.

Un autre objet de l'invention est une matière pulvérulente constituée de particules substrats au moins partiellement enrobées avec au moins un composé (I).
Encore un autre objet de l'invention est l'utilisation d'au moins un composé de formule (I) et/ou d'au moins une matière pulvérulente telle que ci-dessus définie, en tant qu'agent colorant, notamment dans une composition cosmétique.
Un autre objet de l'invention est une composition cosmétique comprenant, dans un milieu cosmétiquement acceptable, au moins une matière pulvérulente telle que ci-dessus définie.

Les composés utilisés dans le cadre de l'invention sont, pour certains, des composés connus dans la littérature. Certains ont notamment été décrits dans la publication "2,2'-Binaphthyliden-1,1'-dione, Farbe und Struktur" de Göltner et al., Liebigs Ann. Chem. 1991, pages 1085-1089. Cette publication décrit notamment un procédé de préparation permettant d'obtenir certains de ces composés sous forme de cristaux de couleur mauve ou bleu, allant du bleu pâle au bleu foncé.
Toutefois, cette publication ne laisse nullement envisager que ces composés peuvent être employés avec succès dans des compositions cosmétiques, c'est-à-dire qu'ils permettent l'obtention d'une composition cosmétiquement acceptable, susceptible d'être appliquée sur la peau, ladite composition ne dégorgeant pas. Il revient à la demanderesse le mérite d'avoir constaté qu'une telle utilisation était possible.

On a également constaté qu'il était en outre possible de moduler la couleur des composés de formule (I) en faisant varier la nature et/ou la position des différents substituants R présents sur la molécule.
On peut ainsi obtenir des composés dont la couleur peut varier du mauve au rouge, en passant par le bleu et le vert.

De plus, les composés utilisés dans le cadre de l'invention présentent une bonne stabilité à la température, au pH et à la lumière.
Ils sont également aisément accessibles par synthèse chimique, même à un niveau industriel.

Les composés utilisés selon l'invention sont donc de formule (I) suivante : 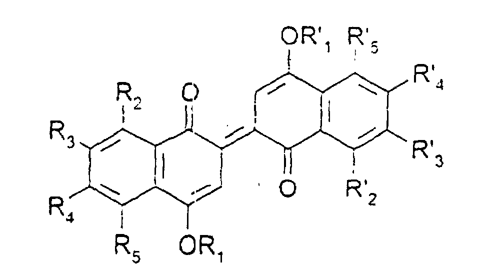 dans laquelle
* R₁ et R'₁ sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes;
* R₂, R'₂, R₃, R'₃, R₄, R'₄, R₅, R'₅ sont, indépendamment les uns des autres, choisis parmi un atome d'hydrogène, un radical halogéné, un radical hydroxyle, un radical alkyle, alkyloxy, acyle ou acyloxy, linéaire ou ramifié, saturé ou insaturé, ayant 1 à 6 atomes de carbone.

Parmi les hétéroatomes, on peut citer les atomes d'oxygène, de silicium, d'azote ou de soufre (O, Si, N ou S).
De préférence, R₁ et/ou R'₁ sont des radicaux alkyles ayant 1 à 8 atomes de carbone, et notamment des radicaux méthyle, éthyle, propyle. isopropyle, butyle, pentyle ou hexyle, Préférentiellement, R₁ et R'₁ représentent le même radical.
De préférence, R₂ à R₅ et R'₂ à R'₅ représentent un atome d'hydrogène.

En particulier, on peut citer comme composés susceptibles d'être utilisés selon l'invention les composés suivants :
- le 4,4'-di-méthyloxy-[2,2']-binaphthylidène-1,1'-dione,
- le 4,4'-di-éthyloxy-[2,2']-binaphthylidène-1,1'-dione,
- le 4,4'-di-isopropyloxy-[2,2']-binaphthylidène-1,1'-dione, et
- le 4,4'-di-n-hexyloxy-[2,2']-binaphthylidène-1,1'-dione,

Les composés de formule (I) se présentent sous forme solide. Ils produisent des couleurs vives et variées, selon la nature des substituants.
Ils sont généralement insolubles dans l'eau et très peu solubles dans des huiles de nature et /ou de polarité variées. En conséquence, ces composés présentent l'avantage de très peu dégorger lorsqu'ils sont utilisés dans des compositions comprenant des corps gras.
Les composés selon l'invention peuvent être aisément préparés par l'homme du métier sur la base de l'art antérieur et de ses connaissances techniques générales.

Les composés de formule (I) peuvent être incorporés dans une composition, notamment cosmétique, en une quantité déterminable aisément par l'homme du métier sur la base de ses connaissances générales, et qui peut notamment être comprise entre 0,1 à 80% en poids par rapport au poids total de la composition, de préférence en une quantité de 0.5 à 70% en poids, plus préférentiellement en une quantité de 1 à 50% en poids, et par exemple en une quantité de 2 à 20% en poids.

Lesdits composés peuvent être présents dans, la composition sous forme libre ou sous forme d'une association avec des particules substrats qu'ils enrobent.

En effet, on a constaté que les composés de formule (I) présentent la particularité de pouvoir enrober, au moins partiellement voire totalement, des particules substrats telles que des pigments ou des charges usuelles.
Parmi les particules susceptibles d'être enrobées par les composés de formule (I), on peut citer notamment les pigments ou nanopigments d'oxydes métalliques comme les oxydes de titane, de zinc. de fer, de manganèse, de césium et/ou de zirconium; les charges blanches telles que le talc, le mica, la silice, le kaolin, les poudres de Nylon et de polyéthylène: les microsphères telles que les microsphères creuses de copolymères de chlorure de vinylidène/acrylonitrile.
De préférence, on choisit le talc comme particule substrat à enrober.

Les matières pulvérulentes ainsi obtenues, constituées des particules substrats enrobées, peuvent alors elles-mêmes être utilisées comme agent colorant dans les compositions notamment cosmétiques.
On a constaté que lesdites matieres pulvérulentes présentent, en microscopie électronique, une structure très homogène.
On a également constaté que lesdites matières pulvérulentes peuvent présenter les propriétés et avantages de chacun des matériaux de départ: en particulier, lorsque l'on enrobe du talc, connu pour apporter de la douceur, on obtient un talc enrobé qui conserve sa douceur.
De plus, l'utilisation d'une très faible quantité de composé de formule (I) enrobant une charge usuelle, permet l'obtention d'un agent colorant ayant une force colorante importante, même utilisé en petite quantité, et apportant une couvrance adéquate ainsi qu'une couleur ayant une bonne tenue à la lumière.
De préférence, on utilisera les composés de formule (I) en une quantité de 0.1 à 100 parties en poids pour 100 parties en poids de particules substrats à enrober.
De préférence, la matière pulvérulente enrobée est constituée de 1 à 50% en poids de composés de formule (I) et de 50 à 99% en poids de particules substrats.
De préférence, la matière pulvérulente enrobée est constituée de 1 à 20% en poids de composés de formule (I) et de 80 à 99% en poids de particules substrats. Toutefois, il est possible d'envisager une matière pulvérulente enrobée constituée de 50% en poids de composés de formule (I) et de 50% en poids de particules substrats.

D'une manière générale, on préparera la matière pulvérulente constituée de particules substrats au moins partiellement enrobées avec le composé de formule (I) de la manière suivante :
- dans un premier temps, on solubilise le composé de formule (I) dans un solvant adéquat, par exemple le DMF.
- puis on précipite ledit composé sur la particule substrat à enrober, par exemple en ajoutant la solution dudit composé à une dispersion ou suspension aqueuse de ladite particule.
On peut ensuite filtrer, laver et sécher la matière pulvérulente selon les techniques classiques.

Lesdits composés de formule (I) et/ou lesdites matières pulvérulentes peuvent être utilisés, notamment comme agent colorant. dans une composition cosmétique qui peut se présenter sous la forme d'un produit à appliquer sur les muqueuses, les semi-muqueuses et/ou les tissus kératiniques tels que la peau et les phanères (ongles, cils, sourcils, poils et cheveux), Ladite composition contient donc un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage. Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libire, compacte ou coulée; une pâte anhydre. L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.
Ladite phase aqueuse peut comprendre de 0 % à 14 % en poids, par rapport au poids total de la phase aqueuse, d'un monoalcool inférieur en C₂-C₆ et/ou d'un polyol tel que le glycérol, le butylèneglycol, l'isoprène glycol, le propylèneglycol, le polyéthylèneglycol.
Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids. par rapport au poids total de la composition, choisis parmi:
- les biopolymères polysaccharidiques comme la gomme de xanthane, la gomme de caroube, la gomme de guar, les alginates, les celluloses modifiées, les dérivés de l'amidon, les dérivés d'éthers de cellulose possédant des groupes ammonium quaternaires, les polysaccharides cationiques;
- les polymères synthétiques comme les acides polyacryliques. la polyvinylpyrrolidone, l'alcool polyvinylique, les polymères à base de polyacrylamide
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les butyralpolyvinyliques, les résines alkydes, les polyesters, les acryliques, les vinyliques. et/ou les polyuréthanes.
La composition peut également comprendre au moins un plastifiant, qui peut être présent à une teneur allant de 1 % à 40 % en poids par rapport au poids total de la composition.

La composition selon l'invention peut également comprendre une phase grasse, notamment constituée de corps gras liquides à 25°C. tels que des huiles d'origine animale, végétale, minérale ou synthétique; de corps gras solides à 25°C tels que des cires d'origine animale, végétale, minérale ou synthétique: de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant. à 25°C, une tension de vapeur saturante au moins égale à 0,5 millibar (soit 50 Pa).

On peut ainsi citer les huiles siliconées volatiles, telles que :
- les silicones volatiles cycliques ayant de 3 à 8 atomes de silicium et de préférence de 4 à 6.
- les cyclocopolymères du type diméthylsiloxane/méthylalkylsiloxane,
- les silicones volatiles linéaires ayant de 2 à 9 atomes de silicium.
   On peut également citer les huiles volatiles hydrocarbonées, telles que les isoparaffines et notamment l'isododécane et des huiles fluorées.

On peut également utiliser des huiles non volatiles, parmi lesquelles on peut citer:
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule : 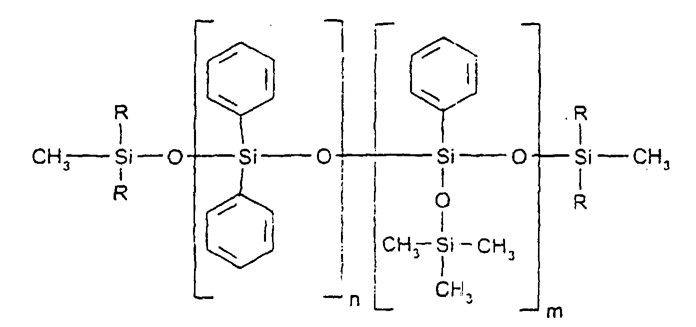 dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.
- les huiles d'origine animale, végétale ou minérale, et notamment les huiles animales ou végétales formées par des esters d'acide gras et de polyols, en particulier les triglycérides liquides, par exemple les huiles de tournesol, de maïs. de soja, de courge, de pépins de raisin, de sésame, de noisette, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras: des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.
On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila. d'Ouricury, de Carnauba, du Japon. le beurre de cacao, les cires de fibres de lièges ou de canne à sucre; les huiles hydrogénées concrètes à 25°C. les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition et peuvent être choisis dans le groupe constitué par les solvants organiques hydrophiles, les solvants organiques lipophiles, les solvants amphiphiles ou leurs mélanges.

La composition peut comprendre en outre une phase particulaire, qui peut comprendre des pigments et/ou des nacres et/ou des charges habituellement utilisés dans les compositions cosmétiques. Par pigments, il faut comprendre des particules blanches ou colorées, minérales ou organiques. destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc. de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium. de baryum, d'aluminium ou de zirconium, de colorants acides.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids. de préférence 5 à 15%, dans la composition, peuvent être minérales ou de synthèse, lamellaires ou non lamellaires. On peut citer le talc. le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba. par exemple), le carbonate de calcium précipité, le carbonate ou l'hydrocarbonate de magnésium, des savons métalliques dérivés d'acides organiques carboxyliques ayant de 8 à 22 atomes de carbone.
Selon le type de formulation, la phase pulvérulente peut représenter de 0,01 à 99% en poids de la composition.
La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des acides gras essentiels, des sphingolipides, des agents autobronzants tels que la dihydroxyacétone (DHA), des filtres solaires, des agents antimousses, des agents séquestrants, des antioxydants.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques selon l'invention peuvent se présenter sous la forme d'un produit de soin et/ou de maquillage de la peau, un produit solaire ou autobronzant, un produit capillaire.

En particulier, elles trouvent une application particulière dans le domaine du maquillage, notamment comme rouges à lèvres, fonds de teint, fards à joues ou à paupières, poudres libres ou compactes, eye-liners, mascaras ou vernis à ongles.

L'invention est illustrée plus en détail dans les exemples suivants.

### Exemple 1 : Préparation du 4.4'-di-méthyloxy-[2,2']-binaphthylidène-1,1'-dione

On dissout, à température ambiante, 10 g de 4-méthoxy-1-naphtol dans un mélange de 1 litre de diméthylacétamide, 250 ml de méthanol et 150 ml d'eau.
On ajoute, sous agitation, 31 g (2 équivalents) de chlorure ferrique hexahydraté. On laisse agiter pendant 5 minutes, puis on filtre le précipité bleu sur verre fritté. On lave et sèche le composé de manière usuelle.
On obtient 5,2 g (rendement : 52%) du pigment recherché, sous forme d'une poudre amorphe bleu/violet.
Point de fusion : 274°C
HPCCM (CH₂Cl₂) : profil monotache Rf = 0,7
HPLC : profil monopic
Spectres de masse, RMN et UV : conformes à la structure attendue Analyse élémentaire :

| | C | H | O |
|---|---|---|---|
| théorique | 76,73 | 4.68 | 18,20 |
| expérimental | 76.94 | 4,64 | 18,53 |

On teste la stabilité de ce composé et l'on obtient les résultats suivants :
- stabilité à 90°C : au moins 16 heures
- stabilité à 45°C : au moins 1 mois
- stabilité à pH 4 : au moins 1 mois
- stabilité à pH 10 : au moins 1 mois
- stabilité à la lumière (Suntest) : au moins 36 heures.

### Exemple 2 : Préparation du 4,4'-di-éthyloxy-[2,2']-binaphthylidène-1,1'-dione

On dissout, à température ambiante, 20 g de 4-éthoxy-1-naphtol dans 500 ml de chloroforme.
On ajoute, sous agitation, 19 g d'oxyde d'argent et on laisse agiter pendant une heure. On filtre le précipité sur verre fritté, on le lave avec du dichlorométhane bouillant jusqu'à décoloration du solvant, on concentre la phase organique et on obtient des cristaux de couleur violet foncé, qui sont séchés.
On obtient 10 g (rendement : 50%) du pigment recherché, sous forme de cristaux.

Point de fusion : 244°C
HPCCM (CH₂Cl₂) : profil monotache Rf = 0,8
Spectre RMN : conforme à la structure attendue
Analyse élémentaire :

| | C | H | O |
|---|---|---|---|
| théorique | 77,40 | 5,41 | 17,18 |
| expérimental | 77,46 | 5,30 | 17,03 |

### Exemple 3 : Préparation du 4,4'-di-isopropyloxy-[2,2']-binaphthylidène-1,1'-dione

On procède de manière similaire à l'exemple 2, à partir de 20 g de 4-isopropyl-1-naphtol et de 20 g d'oxyde d'argent.
On obtient 16,4 g de cristaux violet (rendement ; 83%).

Point de fusion : 230°C
HPCCM (dichlorométhane 8 / heptane 2) : profil monotache Rf = 0,5
Spectre RMN : conforme à la structure attendue
Analyse élémentaire :

| | C | H | O |
|---|---|---|---|
| théorique | 77,98 | 6,04 | 15,98 |
| expérimental | 78,41 | 6,10 | 15,58 |

### Exemple 4 : Préparation du 4,4'-di-n-hexyloxy-[2,2']-binaphthylidène-1.1'-dione

On procède de manière similaire à l'exemple 2, à partir de 7 g de 4-n-hexyl-1-naphtol et de 14 g d'oxyde d'argent.
On obtient 6,0 g de cristaux violet (rendement : 86%).

Point de fusion : 146°C
HPCCM (dichlorométhane 4 / heptane 6) : profil monotache Rf = 0,2
Spectre RMN : conforme à la structure attendue

### Exemple 5 : enrobage de talc

On dissout à chaud 0,6 g de 4,4'-di-méthyloxy-[2.2']-binaphthylidène-1,1'-dione dans 100 ml de diméthylformamide. On coule lentement ce mélange sur une suspension vivement, agitée de 20 g de talc dans 200 ml d'eau. On laisse refroidir jusqu'à température ambiante, on filtre sur verre fritté, on lave à l'eau et on sèche. On obtient un pigment de couleur homogène bleu clair, supporté à 3% en poids sur le talc.

### Exemple 6

On dissout à chaud 2,05 g de 4,4'-di-méthyloxy-[2,2']-binaphthylidène-1,1'-dione dans 410 ml de diméthylformamide. On coule lentement ce mélange sur une suspension vivement agitée de 20,5 g de talc dans 820 ml d'eau, On laisse refroidir jusqu'à température ambiante, on filtre sur verre fritté, on lave à l'eau et on sèche. On obtient un pigment de couleur homogène bleu, supporté à 10% en poids sur le talc.

### Exemple 7

On dissout à chaud 4,1 g de 4,4'-di-méthyloxy-[2,2']-binaphthylidène-1,1'-dione dans 820 ml de diméthylformamide. On coule lentement ce mélange sur une suspension vivement agitée de 20,5 g de talc dans 1640 ml d'eau. On laisse refroidir jusqu'à température ambiante, on filtre sur verre fritté, on lave à l'eau et on sèche. On obtient un pigment de couleur homogène bleu foncé, supporté à 20% en poids sur le talc.

### Exemple 8

On prépare un fard à paupières comprenant les ingrédients suivants :
- talc 38 g
- mica 20 g
- oxychlorure de bismuth 8 g
- stéarate de zinc 3 g
- poudre de Nylon 20 g
- composé de l'exemple 1 5 g
- liant gras qsp 100 g

On obtient un fard à paupières bleu.

### Exemple 9

On prépare un mascara comprenant les ingrédients suivants :
- acide stéarique 6 g
- stéarate de glycéryle 3.5 g
- cire d'abeilles 5,5 g
- cire de Carnauba 2 g
- paraffine 7,5 g
- conservateurs qs
- triéthanolamine 3 g
- gomme d'acacia 6 g
- composé de l'exemple 1 5g
- eau qsp 100 g

On obtient un mascara de couleur bleu.

## Revendications

1. Composition cosmétique comprenant, dans un milieu cosmétiquement acceptable, au moins un composé de formule (I): 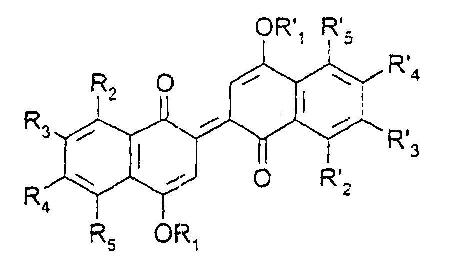 dans laquelle
* R1 et R'1 sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes;
* R2, R'2, R3, R'3, R4, R'4, R5, R'5 sont, indépendamment les uns des autres, choisis parmi un atome d'hydrogène, un radical halogéné, un radical hydroxyle, un radical alkyle, alkyloxy, acyle ou acyloxy, linéaire ou ramifié, saturé ou insaturé, ayant 1 à 6 atomes de carbone.

2. Composition selon la revendication 1, dans laquelle les radicaux R₁ et/ou R'₁ sont des radicaux alkyles ayant 1 à 8 atomes de carbone, et/ou les radicaux R₂ à R₅ et R'₂ à R'₅ représentent un atome d'hydrogène.

3. Composition selon l'une des revendications précédentes, dans laquelle le composé de formule (I) est choisi parmi :
- le 4,4'-di-méthyloxy-[2,2']-binaphthylidène-1,1'-dione,
- le 4,4'-di-éthyloxy-[2,2']-binaphthylidène-1,1'-dione.
- le 4,4'-di-isopropyloxy-[2,2']-binaphthylidène-1,1'-dione, et
- le 4,4'-di-n-hexyloxy-[2,2']-binaphthylidène-1,1'-dione.

4. Composition selon l'une des revendications précédentes, dans laquelle les composés de formule (I) sont présents à raison de 0,1 à 80% en poids, de préférence 0,5 à 70% en poids, par rapport au poids total de la composition.

5. Composition selon l'une des revendications précédentes, dans laquelle les composés de formule (I) sont présents sous forme libre ou sous forme d'une association avec des particules substrats qu'ils enrobent.

6. Composition selon la revendication 5, dans laquelle les particules substrats sont choisies parmi les pigments ou nanopigments d'oxydes métalliques; les charges blanches telles que le talc, le mica, la silice, le kaolin, les poudres de Nylon et de polyéthylène; les microsphères telles que les microsphères creuses de copolymères de chlorure de vinylidène/acrylonitrile.

7. Composition selon l'une des revendications précédentes, dans laquelle le milieu cosmétiquement acceptable comprend ou se présente sous la forme d'une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre.

8. Composition selon l'une des revendications précédentes, se présentant sous la forme d'un produit de soin et/ou de maquillage de la peau, d'un produit solaire ou autobronzant, d'un produit capillaire,

9. Composition selon l'une des revendications précédentes, se présentant sous la forme d'un rouge à lèvres, d'un fond de teint, d'un fard à joues ou à paupières, d'une poudre libre ou compacte, d'un eye-liner, d'un mascara ou d'un vernis à ongles.

10. Composition selon l'une des revendications précédentes, se présentant sous la forme d'un fard à joues ou à paupières.

11. Utilisation d'au moins un composé de formule (I) telle que définie selon l'une quelconque des revendications 1 à 3, pour enrober au moins partiellement des particules substrats.

12. Utilisation selon la revendication 11, dans laquelle les particules substrats sont choisies parmi les pigments et les charges usuelles, et notamment les pigments ou nanopigments d'oxydes métalliques comme les oxydes de titane, de zinc, de fer, de manganèse, de cérium et/ou de zirconium; les charges blanches telles que le talc, le mica, la silice, le kaolin, les poudres de Nylon et de polyéthylène; les microsphères telles que. les microsphères creuses de copolymères de chlorure de vinylidène/acrylonitrile.

13. Matière pulvérulente constituée de particules substrats au moins partiellement enrobées avec au moins un composé de formule (I) telle que definie selon l'une quelconque des revendications 1 à 3.

14. Matière pulvérulente selon la revendication 13, dans laquelle les particules substrats sont choisies parmi les pigments et les charges usuelles, et notamment les pigments ou nanopigments d'oxydes métalliques comme les oxydes de titane, de zinc, de fer, de manganèse, de cérium et/ou de zirconium; les charges blanches telles que le,talc, le mica, la silice, le kaolin, les poudres de Nylon et de polyéthylène; les microsphères telles que les microsphères creuses de copolymères de chlorure de vinylidène/acrylonitrile.

15. Matière pulvérulente selon l'une des revendications 13 à 14, dans laquelle les composés de formule (I) sont présents en une quantité de 0,1 à 100 parties en poids pour 100 parties en poids de particules substrats à enrober.

16. Matière pulvérulente selon l'une des revendications 13 à 15, constituée de 1 à 50% en poids de composés de formule (I) et de 50 à 99% en poids de particules substrats, de préférence de 1 à 20% en poids de composés de formule (I) et de 80 à 99% en poids de particules substrats.

17. Composition cosmétique comprenant, dans un milieu cosmétiquement acceptable, au moins une matière pulvérulente selon l'une des revendications 13 à 16.

18. Composition selon la revendication 17, se présentant sous la forme d'un produit de soin et/ou de maquillage de la peau, d'un produit solaire ou autobronzant, d'un produit capillaire.

19. Composition selon l'une des revendications 17 à 18, se présentant sous la forme d'un rouge à lèvres, d'un fond de teint, d'un fard à joues ou à paupières, d'une poudre libre ou compacte, d'un eye-liner, d'un mascara ou d'un vernis à ongles.

20. Utilisation d'au moins un composé de formule (I) et/ou d'au moins une matière pulvérulente selon l'une des revendications 13 à 16 en tant qu'agent colorant, notamment dans une composition cosmétique.

21. Procédé de préparation d'une matière pulvérulente selon l'une des revendications 13 à 16, dans lequel :
- dans un premier temps, on solubilise le composé de formule (I) dans un solvant adéquat,
- puis on précipite ledit composé sur la particule substrat à enrober.

## Patentansprüche

1. Kosmetische Zusammensetzung, die in einem kosmetisch akzeptablen Medium mindestens eine Verbindung der folgenden Formel (I) enthält: 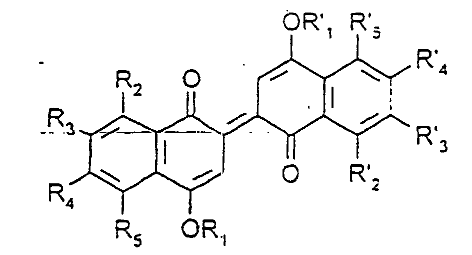 worin:
- R₁ und R'₁ unabhängig voneinander gesättigte oder ungesättigte, geradkettige, verzweigte oder cyclische Alkylgruppen mit 1 bis 18 Kohlenstoffatomen bedeuten, die gegebenenfalls mit einem oder mehreren Halogenen und/oder mit einer oder mehreren Hydroxygruppen substituiert und/oder durch ein oder mehrere Heteroatome unterbrochen sind, und
- R₂, R'₂, R₃, R'₃, R₄, R'₄, R₅ und R'₅ unabhängig voneinander unter einem Wasserstoffatom, einer halogenierten Gruppe, einer Hydroxygruppe, einer Alkylgruppe, einer Alkyloxygruppe, einer Acylgruppe oder einer Acyloxygruppe, die gesättigt oder ungesättigt, geradkettig oder verzweigt ist und 1 bis 6 Kohlenstoffatome aufweist, ausgewählt sind.

2. Zusammensetzung nach Anspruch 1, worin die Gruppen R₁ und/ oder R'₁ Alkylgruppen mit 1 bis 8 Kohlenstoffatomen sind und/oder die Gruppen R₂ bis R₅ und R'₂ bis R'₅ ein Wasserstoffatom bedeuten.

3. Zusammensetzung nach einem der vorhergehenden Ansprüche, worin die Verbindung der Formel (I) ausgewählt ist unter:
- 4,4'-Dimethyloxy-[2,2']-binaphthyliden-1,1'-dion,
- 4,4'-Diethyloxy-[2,2']-binaphthyliden-1,1'-dion,
- 4,4'-Diisopropyloxy-[2,2']-binaphthyliden-1,1'-dion und
- 4,4'-Di-n-hexyloxy-[2,2']-binaphthyliden-1,1'-dion.

4. Zusammensetzung nach einem der vorhergehenden Ansprüche, worin die Verbindungen der Formel (I) in einem Mengenanteil von 0,1 bis 80 Gew.-% und vorzugsweise von 0,5 bis 70 Gew.-%, bezogen auf das Gesamtgewicht der Zusammensetzung, vorliegen.

5. Zusammensetzung nach einem der vorhergehenden Ansprüche, worin die Verbindungen der Formel (I) frei oder in Kombination mit Substratpartikeln, die sie umhüllen, vorliegen.

6. Zusammensetzung nach Anspruch 5, worin die Substratpartikel unter Pigmenten oder Nanopigmenten von Metalloxiden, weißen Füllstoffen, wie Talk, Glimmer, Kieselerde, Kaolin, Nylonpulver und Polyethylenpulver, und Mikrokügelchen, wie hohlen Mikrokügelchen von Vinylidenchlorid/Acrylnitril-Copolymeren, ausgewählt sind.

7. Zusammensetzung nach einem der vorhergehenden Ansprüche, worin das kosmetisch akzeptable Medium eine Suspension, eine Dispersion, eine Lösung in einem Lösemittelmedium oder in einem wässerig-alkoholischen Medium, die gegebenenfalls verdickt oder sogar geliert ist, eine Öl-in-Wasser-Emulsion, eine Wasser-in-Öl-E-mulsion oder eine multiple Emulsion, ein Gel oder einen Schaum, ein emulgiertes Gel, eine Vesikeldispersion und insbesondere eine Lipidvesikeldispersion, eine Zweiphasen- oder Mehrphasenlotion, ein Spray, ein loses, gepresstes oder gegossenes Pulver oder eine wasserfreie Paste enthält oder in diesen Formen vorliegt.

8. Zusammensetzung nach einem der vorhergehenden Ansprüche, die als Pflegeprodukt und/oder Schminkprodukt für die Haut, als Sonnenschutzprodukt oder Selbstbräunungsmittel oder als Produkt zur Haarbehandlung vorliegt.

9. Zusammensetzung nach einem der vorhergehenden Ansprüche, die als Lippenstift, Make-up, Wangenrouge, Lidschatten, loses oder gepresstes Pulver, Eyeliner, Mascara oder Nagellack vorliegt.

10. Zusammensetzung nach einem der vorhergehenden Ansprüche, die als Wangenrouge oder Lidschatten vorliegt.

11. Verwendung mindestens einer Verbindung der Formel (I) nach einem der Ansprüche 1 bis 3, um Substratpartikel zumindest teilweise zu umhüllen.

12. Verwendung nach Anspruch 11, worin die Substratpartikel unter herkömmlichen Pigmenten und Füllstoffen und insbesondere unter Pigmenten oder Nanopigmenten von Metalloxiden, wie den Oxiden von Titan, Zink, Eisen, Mangan, Zerium und/oder Zirconium, weißen Füllstoffen, wie Talk, Glimmer, Kieselerde, Kaolin, Nylonpulver und Polyethylenpulver, und Mikrokügelchen, wie hohlen Mikrokügelchen von Vinylidenchlorid/Acrylnitril-Copolymeren, ausgewählt sind.

13. Pulverförmiger Stoff, der aus Substratpartikeln besteht, die zumindest teilweise mit mindestens einer Verbindung der Formel (I) nach einem der Ansprüche 1 bis 3 umhüllt sind.

14. Pulverförmiger Stoff nach Anspruch 13, worin die Substratpartikel unter herkömmlichen Pigmenten und Füllstoffen und insbesondere unter Pigmenten oder Nanopigmenten von Metalloxiden, wie den Oxiden von Titan, Zink, Eisen, Mangan, Zerium und/oder Zirconium, weißen Füllstoffen, wie Talk, Glimmer, Kieselerde, Kaolin, Nylonpulver und Polyethylenpulver, und Mikrokügelchen, wie hohlen Mikrokügelchen von Vinylidenchlorid/Acrylnitril-Copolymeren, ausgewählt sind.

15. Pulverförmiger Stoff nach einem der Ansprüche 13 bis 14, worin die Verbindungen der Formel (I) in einem Mengenanteil von 0,1 bis 100 Gewichtsteilen auf 100 Gewichtsteile der zu umhüllenden Substratpartikel vorliegen.

16. Pulverförmiger Stoff nach einem der Ansprüche 13 bis 15, der aus 1 bis 50 Gew.-% von Verbindungen der Formel (I) und 50 bis 99 Gew.- % Substratpartikeln, vorzugsweise aus 1 bis 20 Gew.-% von Verbindungen der Formel (I) und 80 bis 99 Gew.-% Substratpartikeln besteht.

17. Kosmetische Zusammensetzung, die in einem kosmetisch akzeptablen Medium mindestens einen pulverförmigen Stoff nach einem der Ansprüche 13 bis 16 enthält.

18. Zusammensetzung nach Anspruch 17, die als Pflegeprodukt und/ oder Schminkprodukt für die Haut, als Sonnenschutzprodukt oder Selbstbräunungsmittel oder als Produkt zur Haarbehandlung vorliegt.

19. Zusammensetzung nach einem der Ansprüche 17 bis 18, die als Lippenstift, Make-up, Wangenrouge, Lidschatten, loses oder gepresstes Pulver, Eyeliner, Mascara oder Nagellack vorliegt.

20. Verwendung mindestens einer Verbindung der Formel (I) und/oder mindestens eines pulverförmigen Stoffs nach einem der Ansprüche 13 bis 16 als Färbemittel insbesondere in einer kosmetischen Zusammensetzung.

21. Verfahren zur Herstellung eines pulverförmigen Stoffs nach einem der Ansprüche 13 bis 16, worin:
- in einem ersten Schritt die Verbindung der Formel (I) in einem geeigneten Lösemittel solubilisiert wird und
- anschließend die Verbindung auf dem zu umhüllenden Substratpartikel gefällt wird.

## Claims

1. Cosmetic composition comprising, in a cosmetically acceptable medium, at least one compound of formula (I): 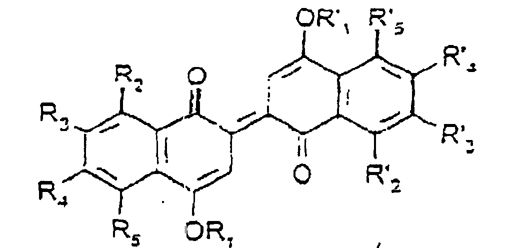 in which
* R₁ and R'₁ are, independently of one another, saturated or unsaturated, linear, branched or cyclic alkyl radicals having 1 to 18 carbon atoms which are optionally substituted by one or more halogens and/or by one or more hydroxyl radicals and/or interrupted by one or more heteroatoms;
* R₂, R'₂, R₃, R'₃, R₄, R'₄, R₅ and R'₅ are, independently of one another, chosen from a hydrogen atom, a halogenated radical, a hydroxyl radical or a saturated or unsaturated, linear or branched, alkyl, alkyloxy, acyl or acyloxy radical having 1 to 6 carbon atoms.

2. Composition according to Claim 1, in which the R₁ and/or R'₁ radicals are alkyl radicals having 1 to 8 carbon atoms and/or the R₂ to R₅ and R'₂ to R'₅ radicals represent a hydrogen atom.

3. Composition according to either of the preceding claims, in which the compound of formula (I) is chosen from:
- 4,4'-dimethyloxy-[2,2'-binaphthylidene]-1,1'-dione,
- 4,4'-diethyloxy-[2,2'-binaphthylidene]-1,1'-dione,
- 4,4'-diisopropyloxy-[2,2'-binaphthylidene]-1,1'-dione, and
- 4,4'-di(n-hexyloxy)-[2,2'-binaphthylidene]-1,1'-dione.

4. Composition according to one of the preceding claims, in which the compounds of formula (I) are present in a proportion of 0.1 to 80% by weight, preferably 0.5 to 70% by weight, with respect to the total weight of the composition.

5. Composition according to one of the preceding claims, in which the compounds of formula (I) are present in the free form or in the form of a combination with substrate particles, which they coat.

6. Composition according to Claim 5, in which the substrate particles are chosen from metal oxide pigments or nanopigments; white fillers, such as talc, mica, silica, kaolin, or nylon and polyethylene powders; or microspheres, such as hollow microspheres formed of vinylidene chloride/acrylonitrile copolymers.

7. Composition according to one of the preceding claims, in which the cosmetically acceptable medium comprises or is provided in the form of a suspension, a dispersion or a solution in solvent or aqueous/alcoholic medium which is optionally thickened, indeed even gelled; an oil-in-water, water-in-oil or multiple emulsion; a gel or a foam; an emulsified gel; a dispersion of vesicles, in particular lipid vesicles; a two-phase or multiphase lotion; a spray; a loose, compact or cast powder; or an anhydrous paste.

8. Composition according to one of the preceding claims, which is provided in the form of a care and/or make-up product for the skin, of an antisun or self-tanning product, or of a hair product.

9. Composition according to one of the preceding claims, which is proviced in the form of a lipstick, of a foundation, of a face powder, of an eyeshadow, of a loose or compact powder, of an eyeliner, of a mascara or of a nail varnish.

10. Composition according to one of the preceding claims, which is provided in the form of a face powder or of an eyeshadow..

11. Use of at least one compound of formula (I) as defined according to any one of Claims 1 to 3 for at least partially coating substrate particles.

12. Use according to Claim 11, in which the substrate particles are chosen from conventional pigments and fillers and in particular metal oxide pigments or nanopigments, such as titanium, zinc, iron, manganese, cerium and/or zirconium oxides; white fillers, such as talc, mica, silica, kaolin, or nylon and polyethylene powders; or microspheres, such as hollow microspheres formed of vinylidene chloride/acrylonitrile copolymers.

13. Pulverulent material composed of substrate particles which are at least partially coated with at least one compound of formula (I) as defined according to any one of Claims 1 to 3.

14. Pulverulent material according to Claim 13, in which the substrate particles are chosen from conventional pigments and fillers and in particular metal oxide pigments or nanopigments, such as titanium, zinc, iron, manganese, cerium and/or zirconium oxides; white fillers, such as talc, mica, silica, kaolin, or nylon and polyethylene powders; or microspheres, such as hollow microspheres formed of vinylidene chloride/acrylonitrile copolymers.

15. Pulverulent material according to either of Claims 13 and 14, in which the compounds of formula (I) are present in an amount of 0.1 to 100 parts by weight per 100 parts by weight of substrate particles to be coated.

16. Pulverulent material according to one of Claims 13 to 15, composed of 1 to 50% by weight of compounds of formula (I) and of 50 to 99% by weight of substrate particles, preferably of 1 to 20% by weight of compounds of formula (I) and of 80 to 99% by weight of substrate particles.

17. Cosmetic composition comprising, in a cosmetically acceptable medium, at least one pulverulent material according to one of Claims 13 to 16.

18. Composition according to Claim 17, which is provided in the form of a care and/or make-up product for the skin, of an antisun or self-tanning product, or of a hair product.

19. Composition according to either of Claims 17 and 18, which is provided in the form of a lipstick, of a foundation, of a face powder, of an eyeshadow, of a loose or compact powder, of an eyeliner, of a mascara or of a nail varnish.

20. Use of at least one compound of formula (I) and/or of at least one pulverulent material according to one of Claims 13 to 16 as colouring agent, in particular in a cosmetic composition.

21. Process for the preparation of a pulverulent material according to one of Claims 13 to 16, in which:
- in a first step, the compound of formula (I) is dissolved in an appropriate solvent,
- then the said compound is precipitated on the substrate particle to be coated.
